# EUROPEAN PATENT APPLICATION

(11) **EP 3 272 368 A1**
(43) Date of publication of application: **24.01.2018**
(21) Application number: 17181557.4
(22) Date of filing: 14.07.2017
(51) Int. Cl.: A61L 9/12

(54) **SCENTED CONTAINER**

(30) Priority: 20.07.2016 CN 201620775580 U
(71) Applicant: Lam, Chan Va, Tianjin (CN)
(72) Inventor: Lam, Chan Va, Tianjin (CN)
(74) Representative: Bailey, David Martin

(57) **Abstract**

The invention relates to a novel structure of scented sheet comprising an outer layer of tin protective film (10) and an inner layer of nanometer film (20) with a folding line (40) formed there between, and a scented sheet (30) is contained in a packing case (100). The scented sheet of the present utility model employs a double-layer isolation technology, i.e., outer layer isolation with tin sheet and inner layer isolation with nanometer film. The outer layer of tin sheet isolates odor so as to prevent the scented sheet from volatizing, while the inner layer of nanometer film isolates liquid rather than prevents odor from volatizing.

## Description

### Technical field

The present invention relates to a scented container or sheath.

### The prior art

In particular, the present invention is concerned with fragrancing containers for air-freshener devices, especially those including a fan or heater to aid dispersion of the fragrance. In such devices, the fragrance is often formed as a gel or as an open container. Thus the scented sheet or container is not well isolated or sealed and the fragrance is prone to volatilize, normally at an inconsistent or irregular rate. Thus the structure of scented sheet in the prior art is typically as a single layer.

### The summary of the utility model

The technical problem solved by the present invention is to provide a novel structure of scented sheet in respect to the defects in the prior art.

To solve the technical problem, the technical solution employed by the present invention is to form a novel structure of scented sheet comprising an outer layer of tin protective film and an inner layer of nanometer film with a folding line formed, there between, and a scented sheet is contained in a packing case.

The novel structure of scented sheet according to the present invention is implemented with the following beneficial effects: the package of the scented sheet of the present invention employs a double-layer isolation technology, i.e., outer layer isolation with tin sheet and inner layer isolation with nanometerfilm. The outer layer of tin sheet isolates odor so as to prevent the scented sheet from volatizing, while the inner layer of nanometer film isolates liquid rather than prevents odor from volatizing.

### Description of the Figure

The above and other aspects of the present invention willnow bedescribed in further detail by reference to the embodiment shown in the accompanying figure, in which:.

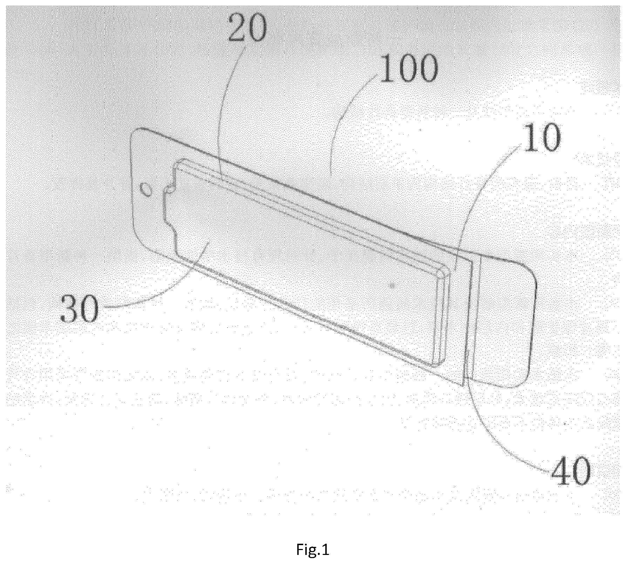

Fig.1 is a structural schematic of the novel structure of an embodiment of a scented sheet according to the present invention.

### The detailed embodiment

In order to clarify the purpose, technical solution and advantage of the present invention, a detailed description of the present invention will be given by embodiment in connection with the Figure. It should be understood that, the detailed embodiment described herein is merely for explanation, but not limitation of the present invention.

Fig.1 is a perspective view of an embodiment of a fragrance pouch or scented sheet 100 of the present invention. The pouch comprises a tray 30 suitably injection or blow-moulded from a plastics material. The desired fragrance is provided as a liquid, suitably a solution in a solvent, in the tray and the tray is provided with a nanometer film 20 thereover. Nanometer film 20 provides a seal to the tray and is selected such that the solvent cannot pass through the pores of the nanometer film 20 but the fragrancing components of the solution are able to pass through, to fragrance a room, for example. A metalised polymeric film 10, or other suitable protective or sealing film is applied to the nanometer film 20 to provide a complete seal to the fragrance pouch, for the purposes of transportation and storage. A fold line 40 is provided in a conventional manner to assist in removal of the protective metalised film 10 from the pouch. The procedure of using it is as follows: unpacking the packing case, separating the scented sheet and its outer layer of tin or metalised protective film along the folding line, checking whether the sealing of the scented sheet package is well, using it with a cover according to the sealing condition.

The package of the scented sheet of the present invention employs a double-layer isolation technology, i.e., outer layer isolation with metalised sheet and inner layer isolation with nanometer film. The outer layer of tin sheet isolates odor so as to prevent the scented sheet from volatizing, while the inner layer of nanometer film isolates liquid rather than prevents fragrance from volatizing.

Although the present invention is disclosed by virtue of embodiment above, the protective scope of the present invention is not limited thereto. Without departing from the spirit of the present invention, any modification and substitute to each part above will fall into the claimed scope of the present invention.

## Claims

1. A fragrance pouch or scented sheet (100) comprising a tray or sheet (30) containing a fragrance solution; a nanometer film (20) applied to the tray or sheet (30) and a protective film (10) applied to the nanometer film.

2. A fragrance pouch or scented sheet as claimed in claim 1, wherein the protective film (10) is a metalised polymeric film.

3. A fragrance pouch or scented sheet as claimed in claim 1 or claim 2, further comprising a fold line (40) to at least the protective film to aid removal of the protective film from the pouch or sheet.

4. A fragrance pouch or scented sheet as claimed in any preceding claim, wherein the fragrance solution includes a solvent and the nanometer film (20) is selected to prevent or minimize transfer of the solvent from the tray or sheet (30).
